# EUROPEAN PATENT APPLICATION

(11) **EP 4 797 273 A1**
(43) Date of publication of application: **26.08.2026**
(21) Application number: 26157562.5
(22) Date of filing: 10.02.2026
(51) Int. Cl.: G16H 20/10, G16H 40/67, G16H 70/40

(54) **TECHNOLOGY-DRIVEN MEDICATION MANAGEMENT SYSTEM FOR THE SECURE PERSONALIZED ASSOCIATION OF MEDICINES TO PATIENTS**

(30) Priority: 21.02.2025 CH 1602025
(71) Applicant: Medgenie AG, 4103 Bottmingen (CH)
(72) Inventor: Ertl, Andrew, 4103 Bottmingen (CH); Forstner-Dambenois, Michael, 4103 Bottmingen (CH); Gutierrez, Israel, 4103 Bottmingen (CH)
(74) Representative: Prins Intellectual Property AG

(57) **Abstract**

The object of the present invention is to create a technology-driven medication management system and method for the secure dispensing of prescription and other medicines to patients, which in particular tailored to the patient's genetic data, which is easy to use. This is reached by using special medication AR-label (4) fixed to packages of medication products which are designed to be easily scanned by a mobile electronic device's camera module, providing immediate access to essential information such as dosage instructions and known side effects via an external server (1) of a healthcare provider for each patient.

## Description

### TECHNICAL FIELD

The present invention describes a technology-driven medication management system, for the secure personalized association of effects of medicines to patients like human and pets, all kinds of prescribed medications, over the counter medication, food supplements, vitamines and/or vaccines or devices to patients, a mobile electronic device for executing the process steps of the medication management system and a medication AR-label as part of the technology-driven medication management system.

### STATE OF THE ART

Modern medicine is increasingly relying on structured medication concepts to maximize the effectiveness of therapies and minimize risks such as incorrect medication and adverse effects. These concepts are based on a combination of individual patient needs, technological innovations and evidence-based medicine.

A central element of modern medication concepts is personalized medicine. This involves using a patient's genetic, physiological and clinical data to select the right therapy. Pharmacogenomics in particular makes it possible to adapt medication and dosages to the patient's genetic profile. This helps to avoid over- or underdosing and side effects.

Polypharmacy, i.e. taking several medications at the same time, poses a risk, especially for vulnerable like old, obese, cancer patients.

Medication plans and regular reviews by doctors or pharmacists ensure that interactions are recognized at an early stage. Interprofessional cooperation, in which doctors, pharmacists and nursing staff work closely together, further improves safety. However, this is cumbersome and expensive and should be replaced by an easy-to-understand system a system.

Technological aids should be used to prevent medication errors or manage patient expectations of the effects of medication on the patient. Electronic medication plans via smartphone and clinical decision support systems help doctors to identify potential risks such as adverse events based on patients particular pharmacogenomic data. Patients also benefit from digital health apps that allow them to manage their medications alongside their doctors or prescribers.

Overall, the combination of personalized approaches, technological support and close doctor-patient communication helps to minimize medication errors and adverse effects and increase the safety of drug therapy.

Managing medications effectively, especially for patients with complex regimens or unique genetic predispositions, poses challenges. Current systems lack automatization, simplicity, accessibility via smart devices, personalization and rely on generic guidelines, increasing the risk of adverse effects and non-compliance.

Inappropriate use of medications is a top five major cause of death and morbidity in the US and around the world and new solutions are absolutely essential.

Existing medication apps provide basic reminders and prescription tracking for patients. We are still a long way from a reliable personalized, technology-driven medication management method. The known procedures are not user-friendly and are not tailored to the patient.

### DESCRIPTION OF THE INVENTION

The object of the present invention is to create a technology-driven medication management system and method for the secure dispensing and the personalized association of medicines to human patients and pets, which in particular tailored to the patient's pharmacogenomic data, which is easy to use and on the one hand enables producers to easily communicate product relevant information to and on the other hand allows patients and consumers to have a kind of conversation with their doctors or prescribers.

With medicines, medication and medication products all kinds of prescribed medications, over the counter medication, food supplements, herbals, vitamines and/or vaccines, and medical devices are meant in this application.

This system addresses a critical need for personalized, technology-driven medication association and pharmacogenomics of the patient, which can be a human or animals and offers a personalized patient friendly summary of adverse events indirectly by Pharmaceutical and Biotech industry.

By combining genetic data of the patient, pharmaceutical data by a novel special label and real-time connectivity with a server database, it empowers patients and providers to make safer, more informed healthcare decisions tailored to patient individual needs and biology, without constant support from a doctor or pharmacist.

Another objects of the subject matter of the invention are:
- to provide an app running on a mobile electronic device for using the medication management method via the internet and an external server with information,
- the external server which is connectable with the app via the internet, delivering medication advice in real-time to the mobile electronic device and
- a multiplicity of novel labels characterizing pharmaceutical products.

### BRIEF DESCRIPTION OF THE DRAWINGS

Further understanding of various aspects of the invention can be obtained by reference to the following detailed description in conjunction with the associated drawings, which are described briefly below.

It should be noted that in the differently described embodiments, the same parts are provided with the same reference symbols or the same component names, the disclosures contained in the entire description being able to be applied analogously to the same parts with the same reference symbols or the same component symbols.

A preferred exemplary embodiment of the subject matter of the invention is described below in conjunction with the attached drawings.
- FIG. 1: shows a schematic representation of the hardware required to implement the technology-driven medication management system described here.
- Fig. 2a to 2c: show schematically various embodiments of the medication labels.

### DESCRIPTION

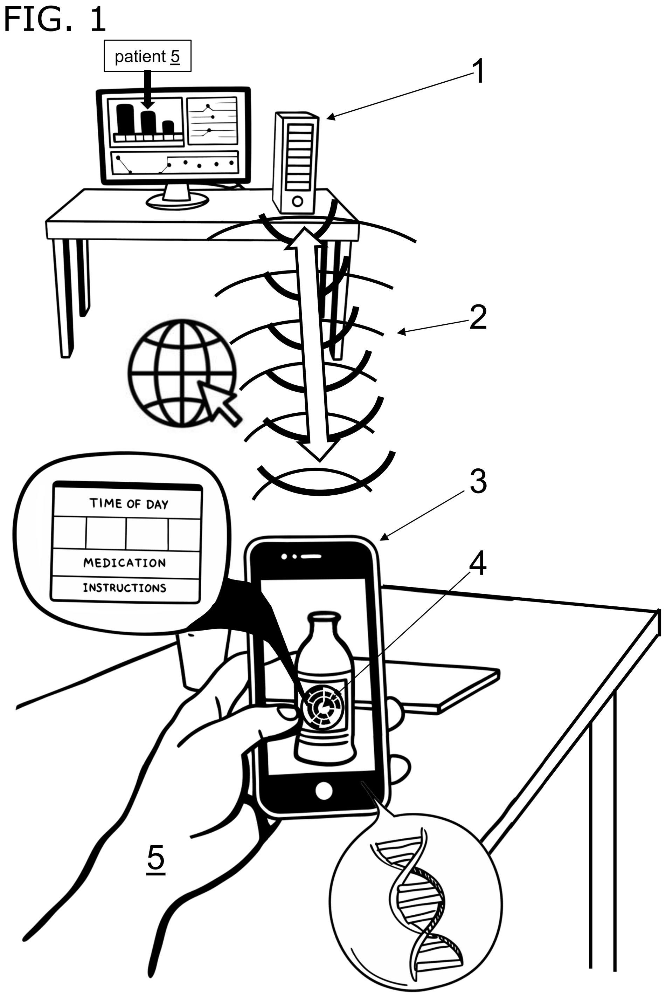

The hardware required for the technology-driven medication management method for the secure dispensing of prescription medicines to patients 5 described here, is shown schematically in Figure 1. Information of medication, i.e. "medical labelling" and "product information" approved by regulatory authorities and/or similar labelling contained on an external server of any method or system provider, medication schemes, adverse events, healthcare risks or medication reminders or any other information relating to a product designed for human or animal consumption or a device are stored on an external server 1. The external server 1 for example of regulatory authorities or the provider of the method respectively system, using data of regulatory authorities, presented here can communicate via a wireless connection or real-time internet connection 2 with mobile electronic device 3.

The wireless and real-time internet connection 2 is designed so that data can be transmitted in both directions simultaneously, i.e. a bidirectional or duplex connection between the external server 1 and the mobile electronic device 3. The external server 1 typically needs a connection unit for establishing the wireless and real-time internet connection 2 to the mobile electronic device 3. Also the external server 1 comprises a database with medication data, adverse effect data in connection with available medication, in particular depending on the patient data, information of tolerance and side effects, due to quantity, concentration and gene dependency. Such information are here independent from pharmacists and physicians and can be communicated to the patient 5 via this method. Patient 5 genetic data is stored on the mobile electronic device 3 only.

The wireless and real-time internet connection 2 between the mobile electronic device 3 and the external remote server 1 are enabled through various technologies. Cellular networks, such as 4G LTE and 5G, or later version provide high-speed, low-latency connections, allowing seamless data exchange with remote servers 1. Wi-Fi offers faster speeds within a limited range, ideal for stable connections in homes or offices. Satellite internet, such as Starlink, ensures connectivity in remote areas where traditional networks are unavailable. Additionally, emerging technologies like LPWAN (Low-Power Wide-Area Network) enable efficient, long-range communication for the wireless and real-time internet connection 2. These wireless and real-time internet connection 2 options ensure real-time data transfer, enabling mobile electronic device 3 to interact with a remote server 1 for tasks like app synchronization, real-time analytics, and cloud computing, enhancing user experiences across diverse environments.

The mobile electronic device 3, such as a smartphone, tablet, or handheld personal computer, a smart watch or smart glasses, equipped with an operating system, serves as the foundation for this medication management system and method. This mobile electronic device 3 hosts a mobile app capable of recognizing medication AR-labels 4 and/or medication name on the packages of medication products. The AR-labels 4 featuring an Augmented Reality (AR) experience. The application interacts with a memory or database on the mobile electronic device 3, which stores patient data, medication details, and personalized genetic information.

By leveraging this database, the mobile electronic device 3 can provide personalized medication recommendations based on a patient's medical history and genetic profile, before or after scanning the medication AR-labels 4 and the medication taken can be saved in the app.

A camera module is integrated into the mobile electronic device 3, enabling it to scan and identify drugs from their medication AR-labels 4. Once a medication is recognized and data was exchanged with the external server 1 via the wireless and real-time internet connection 2, the display module presents relevant information, such as recommended dosages or potential interactions, directly to the patient 5. The processing unit ensures efficient operation by managing data flow, image recognition, and decision-making algorithms.

Users interact with the system through an input module, which allows them to enter personal data, confirm prescriptions, insert genetic data, confirms the use of a medication, or search for specific medications. By combining these components, the system enhances medication safety and improves adherence to prescribed treatments, ensuring personalized and data-driven healthcare decisions. The patient can use the input module of the mobile electronic device 3 to enter additional patient data and the medication actually taken into the database, which optionally will be send to the external server 1 via the wireless connection/real-time internet connection 2.

The medication AR-labels 4 feature an Augmented Reality (AR) experience, showing the additional medical information of this presented method after the medical AR-labels 4 were scanned with the mobile electronic device 3. The medication AR-labels 4 are placed on all kinds of medication products, all kinds of prescribed medications, over the counter medication, food supplements, vitamins, vaccines and and medical devices.

The additional information will be displayed on the display module of the mobile electronic device. The scanned medication AR-label 4 provide quick access to essential information by scanning with most preferred a smartphone 3. Augmented reality (AR) adds a layer of digital interactivity, overlaying crucial details on medication products. Via the medication AR-labels 4 pharmaceutical data of scanned medication can be retrieved via the wireless and real-time internet connection 2 from the external remote server 1.

Preferably, the medication AR-label 4 is generated here as a 3D HUD-style circular hologram graph or Sunburst Chart, which includes sufficient safety and/or medical data and can be scanned. A 3D HUD-style circular hologram graph and/or Sunburst Chart is a powerful visualization technique for representing hierarchical data using concentric radiant data. It consists of multiple rings and layers, with each ring or layer representing a MedDRA hierarchy level. The innermost circle serves as the display of special precaution categories, while the outer rings or layers are divided into segments that illustrate subcategories or detailed data levels. The size and/or color intensity of each pixel segment is proportional to its value or significance within the hierarchy, making it easy to compare different elements. To enhance readability, 3D HUD-style circular hologram graph and Sunburst Charts use color coding to differentiate categories and highlight relationships. These charts are particularly effective for visualizing multi-level hierarchical data, showcasing parent-child relationships, and identifying patterns within complex structures. They provide an intuitive and space-efficient way to analyze large datasets at a glance.

As alternatives for the medication AR-labels 4 a donut chart, a multilayer donut chart or a radial treemap, all types of circular charts used to visualize hierarchical data, can be used. Variation donut charts use different colors or patterns to represent different categories, while multilayer donut charts use multiple rings to show different levels of the hierarchy. Radial treemaps are similar to sunburst charts, but they use color gradients to represent the values of the different categories.

The medication AR-labels 4, which are labeling the packaging of medications, medical devices and/or vaccines can also be created in form of QR codes 4'. QR codes are barcodes that link the medication to digital information. Typically they use a unique pattern of black and white squares to encode text or URLs, enabling quick access to websites, product details, or other online content. The design of the QR code translates visual data into binary, allowing scanners to decode it into readable text.

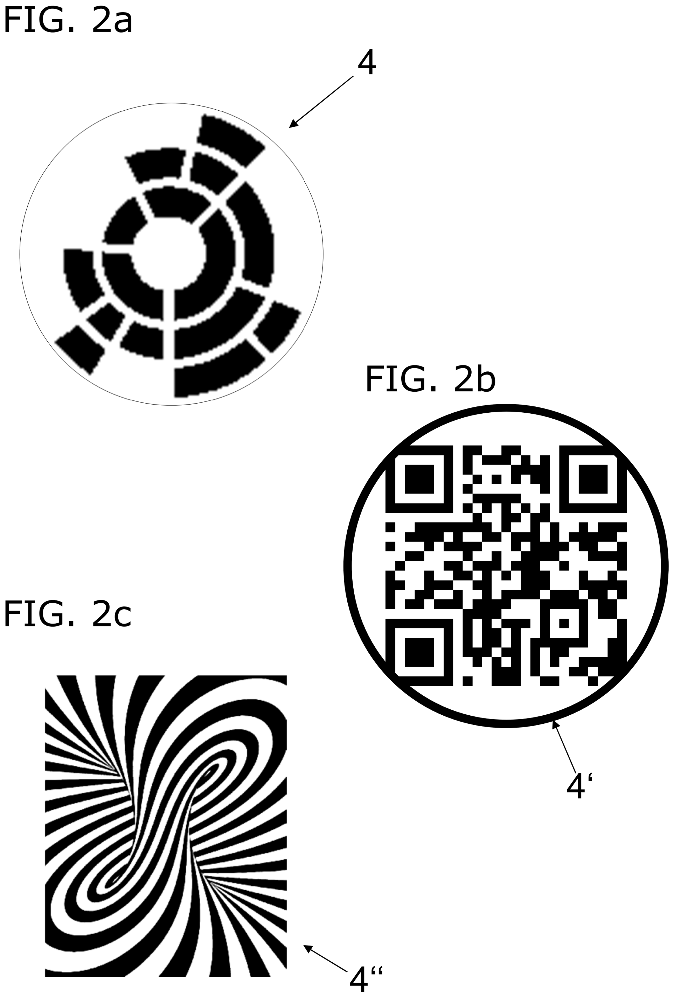

Despite their small size, all mentioned examples from 3D HUD-style circular hologram graph to QR codes can store significant amounts of information efficiently, making them a valuable tool for marketing and daily use. Such QR code medication AR-labels 4' are shown as an example in Fig. 2b.

Another type of medication AR-label 4, 4' is the choice of a hologram as holographic medication AR-label 4", as shown schematically in Fig. 2c. Such holographic medication AR-label 4" are typically combined with line drawings, illustrations, photorealistic images with hyperrealism, macro photography or photomontage, while the person skilled in the art knows the creation of the holographic effect, combined with requests to the external server 1.

All kinds of medication AR-labels 4, 4', 4" enhance patient safety and convenience. Preferably, the medication AR-labels 4, 4', 4" are attached directly to the medical products by adhesive means in the form of stickers. Alternatively, the medication AR-labels 4, 4', 4" can also be printed directly on the medication packaging.

This technology-driven medication management system benefits patients, healthcare providers and producers by increasing compliance, reducing litigation risks, and enhancing safety. By integrating with patient data, such as genetic information, it enables personalized recommendations, ensuring that users receive timely and relevant information. The integration of advanced labeling systems with the mobile electronic device 3 and app not only improves user experience but also strengthens the connection between medication management and individualized healthcare solutions and mitigate at the same time liability and litigation risks of producers.

In the practice, a patient has installed the app on their mobile electronic device 3. By connecting the mobile electronic device 3 to the healthcare provider's external server 1, the patient can be shown medication schedules and even doctor's appointments. The bidirectional data connection between mobile electronic device 3 and external server 1 via real-time internet connection 2 ensures that the patient's secure medication instructions 5 are transmitted.

When the patient has a package of medications, medical devices and/or vaccines in front of him, he scans the medication AR-label 4, 4', 4" attached to or on the package with the camera module of the electronic device 3. After recognizing the image, the mobile electronic device 3 compares data with the external server 3 via the wireless connection 2. The medication to be taken is compared with the schedule and the patient's information and an intake plan, side effects or warnings messages are displayed.

The medication plan is therefore stored on the external server 1 or in the app, tailored to the patient. The dosage instructions are sent via external server 1, wireless connection 2 and the app to the display module for securing possible medication.

In this way, the patient can be made aware to see whether the correct medication is received or a warning about side effects tailored to the patient's pharmacogenomic data. Incorrect intake can thus be virtually ruled out.

If the medication has been taken correctly, the patient can also note this on the mobile electronic device 3 using the input module. The patient's pharmacogenomic data is also advantageously stored, on the mobile electronic device 3 only. For data protection reasons, genetic patient data is only stored on the mobile electronic device 3 respectively in the app. Known side effects are stored on the server and/or in the app for the recognized AR-labels.

The relationship between a patient's pharmacogenomic data and suitable medication is known in science as pharmacogenetics or pharmacogenomics. Pharmacogenetics investigates how individual genetic variations influence a person's response to medication.

The mobile app syncs via the external server 1 with genetic data sources, genomic companies or user genomic data from portable phone, smart phone, personal computer or cloud content to identify potential genetic predispositions to medication-related side effects.

All these processes run automatically in the background after the medication AR-label 4, 4', 4" has been scanned, so that the patient simply has their medication plan displayed on the mobile electronic device 3.

All examples work seamlessly with a mobile electronic device 3, utilizing its camera module to scan and recognize medication AR-labels 4, 4', 4". The app processes these labels, accessing real-time data on known side effects from the server or directly via the app.

The visually presented patient-specific medication schedules and alerts provide improved medication adherence via engaging reminders, reduced healthcare risks with genetically tailored insights and enhanced communication between patients and providers.

Although the invention herein has been described with reference to particular embodiments, it is to be understood that these embodiments are merely illustrative of the principles and applications of the present invention. It will be apparent to those skilled in the art that various modifications and variations can be made to the method and apparatus of the present invention without departing from the spirit and scope of the invention. Thus, it is intended that the present invention include modifications and variations that are within the scope of the appended claims and their equivalents.

Patients 5 could be human and pets. If the system and method is used for pets, of course the pet owner must enter the data and query the medication AR-labels 4 with the mobile electronic device 3.

### LIST OF REFERENCE NUMERALS

1 external Server (run by provider of the method or system or regulatory authorities)
2 wireless connection/ real-time internet connection
3 mobile electronic device (portable or smart phone, tablet, handheld personal computer, smart watch or smart glasses with operating system)
   mobile app, memory/Database (patient data and medication data), Camera module, display module, processor and input module
4 medication AR-label
5 patient (human and pet)

## Claims

1. Technology-driven medication management system, for the secure personalized association of effects of medicines to patients (5) like human and pets, all kinds of prescribed medications, over the counter medication, food supplements, vitamines and/or vaccines or devices to patients (5), comprising:
- an external server (1) of a provider of the method or system or the regulatory authorities, utilized for real-time data access and storage of patient-specific medication scheme, medication information and adverse reaction information in a database on the external server (1) and/or any other product related information contained on such an external server of a method or system provider,
- a wireless connection/real-time internet connection (2) in form of a bidirectional or duplex connection between the external server (1) and
- a mobile electronic device (3), comprising a processor, a operating system, an app, a memory with database, a display module and a camera module, such that the app is capable of recognizing medication AR-labels (4) on packages of medication products, and- the app can send recognized medication AR-label (4) data to the external sever (1) and receive medication information personalized to the patient, while
- in the app's database saved personalized genetic information together with the received personalized information in form of medication recommendations based on patient-specific data are displayed on the display module of the mobile electronic device (3), wherein such elements ensure visually presented patient-specific medication schedules and alerts in real-time for optimal patient care.

2. Technology-driven medication management system according to claim 1, wherein a user and/or the patient (5) can use an input module of the mobile electronic device (3) to enter additional patient data and the medication actually taken into the mobile electronic device (3) database, which optionally will be send to the external server (1) via the wireless connection/real-time internet connection (2).

3. Technology-driven medication management system according to one of the preceding claims, wherein genetic patient data is stored on the mobile electronic device (3) respectively in its a memory with database only.

4. Technology-driven medication management system according to one of the preceding claims, wherein patient's DNA data of the mobile electronic device (3) is mixed up, compared and or merged with product relevant information and medication information on the external server (1), before be send back to the mobile electronic device.

5. Technology-driven medication management system according to one of the preceding claims, wherein the medication AR-label (4) are designed to be scanned by the mobile electronic device's camera module, providing immediate access to essential information such as dosage instructions and known side effects via the external server (1).

6. Technology-driven medication management system according to one of the preceding claims, wherein the medication AR-label (4) are attached directly to the packaging of the medical products by adhesive means in the form of stickers or directly printed on them.

7. Technology-driven medication management system according to one of the preceding claims, wherein the medication AR-label (4, 4', 4") are generated as Sunburst Chart, donut chart, a multilayer donut chart, a radial treemap or as 3D HUD-style circular hologram graph.

8. Technology-driven medication management system according to one of the preceding claims 1 to 6, wherein the medication AR-labels (4) usually prefers the shape of a 3D HUD-style circular hologram graph (4).

9. Technology-driven medication management system according to one of the preceding claims, wherein the wireless and real-time internet connection (2) between the mobile electronic device (3) and the external remote server (1) is based on cellular networks, such as 4G LTE and 5G or later version, a Wi-Fi, WLAN, satellite internet or LPWAN (Low-Power Wide-Area Network) connection.

10. Mobile electronic device (3), such as a smartphone, tablet, or handheld personal computer, a smart watch or smart glasses, as part of the technology-driven medication management system according to one of the preceding claims, providing an operating system, an installed app, a database with patient data, an input module and a camera module, with means for bidirectional or duplex wireless connection (2) between an external server (1) serving as the foundation for the medication management system, wherein the app executes the process steps of the medication management system.

11. Medication AR-label (4) featuring an Augmented Reality (AR) experience, as part of the technology-driven medication management system according to one of the preceding claims 1 to 9, wherein the medication AR-label (4) is capable of featuring an Augmented Reality (AR) experience and is permanently affixed in the form of a sticker on package of medications, medical devices and/or vaccines and is generated as 3D HUD-style circular hologram graph (4).

12. Medication AR-label (4) as part of the technology-driven medication management system according to one of the preceding claims 1 to 9, wherein the medication AR-label (4) is capable of featuring an Augmented Reality (AR) experience and is permanently affixed in the form of a sticker on package of medications and medical devices and is generated as a Sunburst Chart, donut chart, a multilayer donut chart, a radial treemap, a bar code, a QR code or a hologram, indirectly containing safety information of the medication.
